# EUROPEAN PATENT SPECIFICATION

(11) **EP 1 825 855 B1**
(45) Date of publication and mention of the grant of the patent: **17.08.2016**
(21) Application number: 05814731.5
(22) Date of filing: 09.12.2005
(51) Int. Cl.: A61K 31/5575, A61P 27/02, A61L 2/20, A61K 9/08

(54) **PRODUCT CONTAINING PROSTAGLANDIN HAVING FLUORINE ATOM IN MOLECULE**
PROSTAGLANDINHALTIGES PRODUKT MIT FLUORATOM IM MOLEKÜL
PRODUIT CONTENANT DE LA PROSTAGLANDINE DONT LA MOLECULE COMPORTE UN ATOME DE FLUOR

(30) Priority: 09.12.2004 JP 2004356969
(43) Date of publication of application: 29.08.2007
(73) Proprietor: SANTEN PHARMACEUTICAL CO., LTD., Higashiyodogawa-ku, Osaka-shi, Osaka 533-8651 (JP)
(72) Inventor: KADO, Takehiro,, Osaka-shi, Osaka 5338651 (JP); OKAMOTO, Tomoyuki,, Osaka-shi, Osaka 5338651 (JP); ASADA, Hiroyuki,, Osaka-shi, Osaka 5338651 (JP); KIMURA, Akio,, Osaka-shi, Osaka 5338651 (JP)
(74) Representative: Müller-Boré & Partner Patentanwälte PartG mbB
(86) International application number: PCT/JP2005/022618
(87) International publication number: WO 2006/062184

(56) References cited:
- EP-A1- 1 040 840
- EP-A2- 0 850 926
- WO-A1-00/03736
- WO-A1-00/73156
- JP-A- 11 071 344
- JP-A- 2004 516 099
- US-B1- 6 235 781
- HELLBERG M R ET AL: "Unique Polypropylene Packaging System for TRAVATAN(R) Minimizes Loss to the Container" ARVO ANNUAL MEETING ABSTRACT SEARCH AND PROGRAM PLANNER (ANNUAL MEETING OF THE ASSOCIATION FOR RESEARCH IN VISION AND OPHTHALMOLOGY; FORT LAUDERDALE, FLORIDA, USA; MAY 05-10, 2002),, vol. 2002, 5 May 2002 (2002-05-05), pages 2318-20020505, XP009118003

## Description

The present invention relates to a prostaglandin-containing product comprising an aqueous liquid preparation containing a prostaglandin derivative having at least a fluorine atom in its molecule or a salt thereof stored in a resin container subjected to a sterilization treatment, characterized in that a decrease in the content of the prostaglandin derivative in the aqueous liquid preparation is inhibited by performing the sterilization treatment with ethylene oxide gas, and a method for producing the product.

In general, as a container for storing an aqueous liquid preparation, a resin container obtained by molding a synthetic resin such as polypropylene, a propylene/ethylene copolymer or polyethylene is used. Here, the aqueous liquid preparation such as an eye drop is administered directly to the eye which is a particularly sensitive organ in the human body, therefore, it is strictly demanded that a sterile condition should be maintained until it is instilled. In view of this, a container for storing an eye drop has to be in a sterile condition and needs to be subjected to a sterilization treatment. As a sterilization method for such a container, gamma-ray sterilization, electron-ray sterilization, ethylene oxide gas sterilization, hydrogen peroxide solution sterilization, autoclave sterilization and the like are known.

On the other hand, prostaglandin is a physiologically active substance, and a large number of prostaglandin derivatives have been studied and developed. As the prostaglandin useful for ophthalmic applications, a 13,14-dihydro-15-keto-prostaglandin derivative is disclosed in JP-A-2-108, a 13,14-dihydro-17-phenyl-18,19,20-trinor-prostaglandin F2α derivative is disclosed in JP-T-3-501025, a 15-deoxy-15-monofluoro-prostaglandin F2α derivative is disclosed in JP-A-10-251225, and a 15-deoxy-15,15-difluoro-prostaglandin F2α derivative is disclosed in JP-A-11-71344. In terms of the structures of the prostaglandin derivatives, JP-A-2-108 and JP-T-3-501025 are directed to prostaglandin derivatives having no fluorine atom in their molecules, and JP-A-10-251225 and JP-A-11-71344 are directed to prostaglandin derivatives having one or two fluorine atoms in their molecules.

In terms of the storage stability of such a prostaglandin derivative, when 13,14-dihydro-17-phenyl-18,19,20-trinor-prostaglandin F2α isopropyl ester (latanoprost), which is a prostaglandin derivative having no fluorine atom in its molecule, is stored in, for example, a resin container subjected to a sterilization treatment with gamma rays or electron rays, the content of latanoprost does not decrease with time. However, a prostaglandin derivative having at least a fluorine atom in its molecule has a specific property that when it is stored in a resin container subjected to a sterilization treatment with gamma rays or electron rays, the content thereof is liable to decrease.

Therefore, in the case where an aqueous liquid preparation containing a prostaglandin derivative having at least a fluorine atom in its molecule is stored in a resin container, it is an important object to find a sterilization method capable of inhibiting the content of the prostaglandin derivative in the aqueous liquid preparation from decreasing.

The present inventors made intensive studies in order to achieve the above object, and as a result, they found that when an aqueous liquid preparation containing a prostaglandin derivative having at least a fluorine atom in its molecule is stored in a resin container subjected to a sterilization treatment with ethylene oxide, a decrease in the content of the prostaglandin derivative having at least a fluorine atom in its molecule in the aqueous liquid preparation can be significantly inhibited, and thus the present invention has been accomplished.

That is, the present invention relates to:
(1) a prostaglandin-containing product comprising an aqueous liquid preparation containing a prostaglandin derivative having at least a fluorine atom in its molecule or a salt thereof stored in a resin container subjected to a sterilization treatment, characterized in that a decrease in the content of the prostaglandin derivative or a salt thereof in the aqueous liquid preparation is inhibited by performing the sterilization treatment of the container with ethylene oxide gas,
   wherein the prostaglandin derivative having at least a fluorine atom in its molecule is 16-phenoxy-15-deoxy-15, 15-difluoro-17, 18, 19, 20-tetranor-prostaglandin F2α or an alkyl ester thereof, and
   wherein a material of the resin container is a propylene/ethylene copolymer;
(2) the prostaglandin-containing product according to the above (1), wherein the aqueous liquid preparation is an eye drop;
(3) a method for producing a prostaglandin-containing product comprising an aqueous liquid preparation containing a prostaglandin derivative having at least a fluorine atom in its molecule or a salt thereof stored in a resin container subjected to a sterilization treatment, characterized by performing the sterilization treatment of the container with ethylene oxide gas to inhibit the content of the prostaglandin derivative or a salt thereof in the aqueous liquid preparation from decreasing
   wherein the prostaglandin derivative having at least a fluorine atom in its molecule is 16-phenoxy-15-deoxy-15, 15-difluoro-17, 18, 19, 20-tetranor-prostaglandin F2α or an alkyl ester thereof, and
   wherein a material of the resin container is a propylene/ethylene copolymer; and
(4) the method according to the above (3), wherein the aqueous liquid preparation is an eye drop.

The prostaglandin derivative having at least a fluorine atom in its molecule (hereinafter referred to as the present prostaglandin derivative), which is an active ingredient of the prostaglandin-containing product according to the present invention, is 16-phenoxy-15-deoxy- 15,15-difluoro-17,18,19,20-tetranor-prostaglandin F2α, alkyl esters thereof and salts thereof. Specific examples of the alkyl ester include lower alkyl esters such as methyl esters, ethyl esters, propyl esters, isopropyl esters, tert-butyl esters, pentyl esters and hexyl esters.

The resin container subjected to a sterilization treatment with ethylene oxide gas is not particularly limited as long as it is a resin container subjected to a sterilization treatment with ethylene oxide gas.

The ethylene oxide gas sterilization is not particularly limited as long as it is a method of performing a sterilization treatment with ethylene oxide gas, however, for example, it is carried out by the following process. Sterilization is carried out by exposing a resin container to ethylene oxide gas under predetermined temperature and humidity conditions for a time sufficient to effect sterilization, and then, aeration may be carried out for removing ethylene oxide gas. The sterilization temperature can be selected appropriately according to the characteristic of the resin container, however, it is preferably in the range of from 30 to 60°C. Further, the relative humidity is in the range of from 20 to 90%, preferably from 30 to 80%.

The material of the resin container is a propylene/ethylene copolymer. Here, the propylene/ethylene copolymer is not particularly limited as long as it is a propylene polymer containing an ethylene component, however, it is preferably a propylene polymer containing an ethylene component in an amount of 10 mol% or less.

In the prostaglandin-containing product of the present invention, it is preferred that the present prostaglandin derivative exists in the container in a state of being dissolved in water. The concentration of the present prostaglandin derivative can be selected appropriately in view of the application of the aqueous liquid preparation or the like. For example, in the case of an eye drop, the concentration of the present prostaglandin derivative in the eye drop is preferably in the range of from 0.00005 to 0.05% (w/v), although it can be selected appropriately according to the disease to be treated, symptoms or the like.

The content of the present prostaglandin derivative refers to the ratio (%) of the concentration of the present prostaglandin derivative existing in the aqueous liquid preparation after a predetermined period of time to the original concentration of the present prostaglandin derivative existing in the aqueous liquid preparation when the prostaglandin-containing product is produced throughout the claims and the specification. For example, in the case where the present prostaglandin derivative is dissolved in water, it refers to the ratio (%) of the concentration of the present prostaglandin derivative existing in the aqueous solution after a predetermined period of time to the concentration of the present prostaglandin derivative dissolved in water.

When the prostaglandin-containing product of the present invention is an eye drop, a surfactant, an antioxidant, a tonicity agent, a buffer, a preservative or the like can be added thereto as needed. Examples of the surfactant include polysorbate 80, polyoxyethylene hydrogenated castor oil 60, polyoxyl 40 stearate and the like. Examples of the antioxidant include ethylenediamine tetraacetic acid, salts thereof, dibutyl hydroxy toluene and the like. Examples of the tonicity agent include sodium chloride, potassium chloride, calcium chloride, glycerin, propylene glycol and the like. Examples of the buffer include boric acid, borax, citric acid, disodium hydrogenphosphate, ε-aminocaproic acid and the like. Examples of the preservative include benzalkonium chloride, chlorhexidine gluconate, benzethonium chloride, sorbic acid, sodium sorbate, ethyl parahydroxybenzoate, butyl parahydroxybenzoate and the like. A method for preparing the eye drop containing the present prostaglandin derivative does not require a special method or procedure, and the eye drop can be prepared by a widely used method. The pH of the eye drop is preferably adjusted to 3 to 8, particularly preferably 4 to 7.

As will be explained in detail in the section of storage stability test described later, when the aqueous liquid preparation of the present invention containing the present prostaglandin derivative is stored in the resin container subjected to a sterilization treatment with ethylene oxide, a decrease in the content of the present prostaglandin derivative in the aqueous liquid preparation can be significantly inhibited in comparison with the case where it is stored in the resin container treated by either gamma-ray sterilization or electron-ray sterilization.

Hereinafter, the present invention will be described in detail through carrying out a storage stability test. However, such descriptions are disclosed for the purpose of understanding the present invention better and are not meant to limit the scope of the present invention.

### 1. Preparation of Eye Drop

### 1-1. Preparation Method for Eye Drop 1

As a typical example of the present prostaglandin derivative, 0.0005% (w/v) of 16-phenoxy-15-deoxy-15,15-difluoro-17,18,19,20-tetranor-prostaglandin F2α isopropyl ester (hereinafter, referred to as the present compound) was used. The present compound was dissolved in purified water by using polysorbate 80 at 0.05% (w/v) as a nonionic surfactant, and then, additives, which are commonly used in an eye drop, such as ethylenediamine tetraacetic acid (q.s.), concentrated glycerin (q.s.) and benzalkonium chloride (q.s.), and the like were added thereto, whereby Eye drop 1 with an osmotic pressure of about 1 and a pH of about 6 was obtained.

### 1-2. Preparation Method for Eye Drop 2

By using latanoprost which is a prostaglandin derivative having no fluorine atom in its molecule instead of the present compound, Eye drop 2 was obtained by the same procedure as that of the preparation method for Eye drop 1 of item 1-1.

### 2. Production of Resin Container

A 5-ml eye drop resin container was obtained by injection blow molding of a propylene/ethylene random copolymer (containing an ethylene component in an amount of 3%).

### 3. Test Method

### Example 1

The resin container obtained in the section of "2. Production of Resin Container" was subjected to a sterilization treatment with ethylene oxide gas (sterilization with ethylene oxide at a concentration of 20% (v/v), at a temperature of 40°C and a relative humidity of 50% for 3 hours), and then, in the container, Eye drop 1 obtained in the section of "1. Preparation of Eye Drop" was charged. The container containing this eye drop was stored at a temperature of 40°C and a relative humidity of 75% for 30 days. The concentration of the present compound in the container was measured by high performance liquid chromatography before the initiation of storage and after 30-day storage. The content of the present compound was calculated taking the concentration of the present compound at the time of initiation of storage as a standard (100%). The results are shown in Table 1. Incidentally, each value in Table 1 is expressed as an average of three cases.

### Comparative example 1

Except that the sterilization treatment with ethylene oxide gas was altered into a sterilization treatment with gamma rays (32 kGy), the content of the present compound was calculated in the same manner as in Example 1.

### Comparative example 2

Except that the sterilization treatment with ethylene oxide gas was altered into a sterilization treatment with electron rays (23 kGy), the content of the present compound was calculated in the same manner as in Example 1.

### Comparative example 3

Except that a container with no sterilization treatment was used as the container, the content of the present compound was calculated in the same manner as in Example 1.

### Comparative example 4

Except that Eye drop 1 was altered into Eye drop 2, the content of latanoprost was calculated in the same manner as in Example 1.

### Comparative example 5

Except that Eye drop 1 was altered into Eye drop 2 and the sterilization treatment with ethylene oxide gas was altered into a sterilization treatment with gamma rays (32 kGy), the content of latanoprost was calculated in the same manner as in Example 1.

### Comparative example 6

Except that Eye drop 1 was altered into Eye drop 2 and the sterilization treatment with ethylene oxide gas was altered into a sterilization treatment with electron rays (23 kGy), the content of latanoprost was calculated in the same manner as in Example 1.

### Comparative example 7

Except that Eye drop 1 was altered into Eye drop 2 and a container with no sterilization treatment was used as the container, the content of latanoprost was calculated in the same manner as in Example 1.

**[Table 1]**

| | Liquid charged | Sterilization treatment method | Content (%) 30 days |
|---|---|---|---|
| Example 1 | Eye drop 1 | Ethylene oxide gas sterilization | 96.5 |
| Comparative example 1 | Eye drop 1 | Gamma-ray sterilization | 78.4 |
| Comparative example 2 | Eye drop 1 | Electron-ray sterilization | 84.5 |
| Comparative example 3 | Eye drop 1 | No treatment | 96.6 |
| Comparative example 4 | Eye drop 2 | Ethylene oxide gas sterilization | 103.2 |
| Comparative example 5 | Eye drop 2 | Gamma-ray sterilization | 99.4 |
| Comparative example 6 | Eye drop 2 | Electron-ray sterilization | 101.1 |
| Comparative example 7 | Eye drop 2 | No treatment | 103.2 |

### 4. Test Results and Discussion

As is clear from Table 1, when an eye drop containing the present compound was stored in a container made of a propylene/ethylene random copolymer subjected to a sterilization treatment with ethylene oxide gas, the content of the present compound in the eye drop after a predetermined period of time was higher in comparison with the case where it was stored in a container subjected to a sterilization treatment with gamma rays or electron rays. Further, as is clear from Example 1 and Comparative example 3, when an eye drop containing the present compound was stored in a container subjected to a sterilization treatment with ethylene oxide gas, the content of the present compound in the eye drop after a predetermined period of time was comparable even if it was compared with the case with no sterilization treatment. Thus, it was confirmed that when the present compound is stored in a resin container subjected to a sterilization treatment with ethylene oxide gas, a decrease in the present compound in an aqueous liquid preparation can be inhibited.

Further, as is clear from Comparative examples 4 to 7, even if an eye drop containing latanoprost which is a prostaglandin derivative having no fluorine atom in its molecule was stored in a container subjected to a sterilization treatment with gamma rays, electron rays or ethylene oxide gas, the content of latanoprost in the eye drop did not decrease in any of the cases, and moreover, even if it was compared with the case where it was stored in a container with no sterilization treatment, the content of latanoprost in the eye drop was comparable. Thus, it was confirmed that even if an aqueous liquid preparation containing a prostaglandin derivative having no fluorine atom in its molecule such as latanoprost is stored in a container subjected to any sterilization treatment, it is stable.

## Claims

1. A prostaglandin-containing product comprising an aqueous liquid preparation containing a prostaglandin derivative having at least a fluorine atom in its molecule or a salt thereof stored in a resin container subjected to a sterilization treatment, **characterized in that** a decrease in the content of the prostaglandin derivative or a salt thereof in the aqueous liquid preparation is inhibited by performing the sterilization treatment of the container with ethylene oxide gas, wherein the prostaglandin derivative having at least a fluorine atom in its molecule is 16-phenoxy-15-deoxy-15,15-difluoro-17,18,19,20-tetranor-prostaglandin F2α or an alkyl ester thereof, and
wherein a material of the resin container is a propylene/ethylene copolymer.

2. The prostaglandin-containing product according to claim 1, wherein the aqueous liquid preparation is an eye drop.

3. A method for producing a prostaglandin-containing product comprising an aqueous liquid preparation containing a prostaglandin derivative having at least a fluorine atom in its molecule or a salt thereof stored in a resin container subjected to a sterilization treatment, **characterized by** performing the sterilization treatment of the container with ethylene oxide gas to inhibit the content of the prostaglandin derivative or a salt thereof in the aqueous liquid preparation from decreasing,
wherein the prostaglandin derivative having at least a fluorine atom in its molecule is 16-phenoxy-15-deoxy-15,15-difluoro-17,18,19,20-tetranor-prostaglandin F2α or an alkyl ester thereof, and
wherein a material of the resin container is a propylene/ethylene copolymer.

4. The method according to claim 3, wherein the aqueous liquid preparation is an eye drop.

## Patentansprüche

1. Prostaglandinhaltiges Produkt, umfassend eine wässrige, flüssige Zubereitung, enthaltend ein Prostaglandinderivat, das mindestens ein Fluoratom in seinem Molkül aufweist, oder ein Salz davon, aufbewahrt in einem einer Sterilisierungsbehandlung ausgesetzten Harzbehälter, **dadurch gekennzeichnet, dass** eine Verringerung des Gehalts des Prostaglandinderivats oder dessen Salz in der wässrigen, flüssigen Zubereitung durch das Durchführen der Sterilisierungsbehandlung des Behälters mit Ethylenoxidgas gehindert wird, wobei das Prostaglandinderivat, das mindestens ein Fluoratom in seinem Molkül aufweist, 16-Phenoxy-15-deoxy-15,15-difluor-17,18,19,20-tetranor-prostaglandin F2α oder ein Alkylester davon ist, und
wobei ein Material des Harzbehälters ein Propylen/Ethylen-Copolymer ist.

2. Prostaglandinhaltiges Produkt nach Anspruch 1, wobei die wässrige, flüssige Zubereitung Augentropfen ist.

3. Verfahren zur Herstellung eines Prostaglandinhaltigen Produkts, umfassend eine wässrige, flüssige Zubereitung, enthaltend ein Prostaglandinderivat, das mindestens ein Fluoratom in seinem Molkül aufweist, oder ein Salz davon, aufbewahrt in einem einer Sterilisierungsbehandlung ausgesetzten Harzbehälter, **gekennzeichnet durch** das Durchführen der Sterilisierungsbehandlung des Behälters mit Ethylenoxidgas, um den Gehalt des Prostaglandinderivats oder dessen Salz in der wässrigen, flüssigen Zubereitung vor einer Verringerung zu hindern, wobei das Prostaglandinderivat, das mindestens ein Fluoratom in seinem Molkül aufweist, 16-Phenoxy-15-deoxy-15,15-difluor-17,18,19,20-tetranorprostaglandin F2α oder ein Alkylester davon ist, und
wobei ein Material des Harzbehälters ein Propylen/Ethylen-Copolymer ist.

4. Verfahren nach Anspruch 3, wobei die wässrige, flüssige Zubereitung Augentropfen ist.

## Revendications

1. Produit contenant de la prostaglandine comprenant une préparation aqueuse liquide contenant un dérivé de prostaglandine possédant au moins un atome de fluor dans sa molécule ou un sel de celui-ci stocké dans un récipient en résine soumis à un traitement de stérilisation, **caractérisé en ce qu'**une diminution de la teneur du dérivé de prostaglandine ou d'un sel de celui-ci dans la préparation aqueuse liquide est inhibée par la réalisation d'un traitement de stérilisation du récipient avec du gaz d'oxyde d'éthylène, dans lequel le dérivé de prostaglandine possédant au moins un atome de fluor dans sa molécule est 16-phénoxy-15-déoxy-15,15difluoro-17,18,19,20-tétranor-prostaglandine F2α ou un ester d'alkyle de celui-ci, et
dans lequel un matériau du récipient en résine est un copolymère propylène/éthylène.

2. Produit contenant de la prostaglandine selon la revendication 1, dans lequel la préparation aqueuse liquide est une goutte pour les yeux.

3. Méthode de fabrication d'un produit contenant de la prostaglandine comprenant une préparation aqueuse liquide contenant un dérivé de prostaglandine possédant au moins un atome de fluor dans sa molécule ou un sel de celui-ci stocké dans un récipient en résine soumis à un traitement de stérilisation, **caractérisé par** la réalisation du traitement de stérilisation du récipient avec du gaz d'oxyde d'éthylène afin d'inhiber une diminution de la teneur du dérivé de prostaglandine ou un sel de celui-ci dans la préparation aqueuse liquide,
dans laquelle le dérivé de prostaglandine possédant au moins un atome de fluor dans sa molécule est 16-phénoxy-15-déoxy-15,15difluoro-17,18,19,20-tétranor-prostaglandine F2α ou un ester d'alkyle de celui-ci,
et dans laquelle un matériau du récipient en résine est un copolymère propylène/éthylène.

4. Méthode selon la revendication 3, dans laquelle la préparation liquide est une goutte pour les yeux.
